# EUROPEAN PATENT SPECIFICATION

(11) **EP 3 006 075 B1**
(45) Date of publication and mention of the grant of the patent: **09.10.2019**
(21) Application number: 15185388.4
(22) Date of filing: 16.09.2015
(51) Int. Cl.: A61M 25/09

(54) **GUIDEWIRE**
FÜHRUNGSDRAHT
FIL-GUIDE

(30) Priority: 26.09.2014 JP 2014196231
(43) Date of publication of application: 13.04.2016
(73) Proprietor: Asahi Intecc Co., Ltd., Seto-shi, Aichi 489-0071 (JP)
(72) Inventor: SHIMIZU, Eriko, Seto-shi, Aichi 489-0071 (JP); ASHIDA, Shinichi, Seto-shi, Aichi 489-0071 (JP)
(74) Representative: Winter, Brandl, Fürniss, Hübner, Röss, Kaiser, Polte - Partnerschaft mbB

(56) References cited:
- EP-A1- 2 415 497
- EP-A1- 2 586 483
- US-A1- 2005 054 950
- US-A1- 2007 185 415
- US-A1- 2010 023 035

## Description

### TECHNICAL FIELD

The present invention relates to a guide wire to be inserted into a blood vessel or digestive organ for the purpose of treatment or examination.

### BACKGROUND ART

When a narrowed or obstructed part is formed in a blood vessel, bile duct, pancreatic duct and the like, the flow of blood, bile (gall), pancreatic fluid and the like may be hindered. Conventionally, a treatment for improving the flow of blood, bile (gall), pancreatic fluid and the like is widely performed by inserting a guide wire into a narrowed or obstructed part, and then expanding the narrowed or obstructed part with a balloon catheter which follows the guide wire.

Various guide wires intended for guiding a balloon catheter to a narrowed or obstructed part have been proposed. For example, EP 2415497 A1 discloses a guide wire according to the preamble of claim 1, wherein it comprises a core shaft and a coil body wound around an outer periphery of the core shaft. A distal end portion of the core shaft and a distal end portion of the coil body are fixed to each other with a most distal end portion forming a distal end fixed portion. The coil body may be a single wire coil made of a singlestrand, or a multistrand coil made of a plurality of strands.

Further, JP 4354523 B discloses a guide wire comprising a core shaft, a coil body covering a distal end portion of the core shaft and formed of one wound wire and a distal end fixed portion where the distal end of the core shaft is fixed to the distal end of the coil body. JP 2008-155052 A discloses a guide wire comprising a core shaft, a coil body covering a distal end portion of the core shaft and formed of a plurality of spirally wound wires and a distal end fixed portion where the distal end of the core shaft is fixed to the distal end of the coil body.

Further, US 2005 054950 A1 discloses an intracorporeal device which includes a helically wound coil having a plurality of windings covering a distal end portion of an elongate core shaft and forming a coil length, a distal tip forming a distal end fixed portion, and a plurality of joining elements

EP2586483 A1 discloses a guide wire which includes a core shaft and a coil body covering the core shaft disposed along the coil length and coupling a plurality of coil windings together. The guide wire also includes a tip portion forming a distal end fixed portion fixing the core shaft and the coil body to each other.

### TECHNICAL PROBLEM

However, in such guide wires, wires of a coil body are not welded to each other at the distal end of the coil body. Therefore, when fixing the distal end of a core shaft to the distal end of a coil body at a distal end fixed portion, the following problems occur: the distal end fixed portion may flow towards the proximal end side along the surfaces of all of the wires, and thus the length of the distal end fixed portion is difficult to control; the wires of the coil body may be unwound, and thus the distal end fixed portion is difficult to be fixed to the distal end of the coil body.

Further, although it is not a guide wire, known is a catheter in which an end of a coil body formed of spirally wound wires is connected to an end of a braid by welding. In that known catheter, the braid comprises a first wire and a second wire braided in a net-like structure (see JP 2012-192177 A). In a catheter described in JP 2012-192177 A, the wires of the coil body are melted together to fix the wires each other in order to improve the connectivity between a coil body and a braid.

When the coil body disclosed in JP 2012-192177 A is applied to the above guide wire, the flow of the distal end fixed portion towards the proximal end side along the wires can be prevented, and further the distal end fixed portion can be easily fixed to the distal end of the coil body. However, the tensile strength of the distal end fixed portion becomes weak, resulting in a risk that the distal end fixed portion falls off from the distal end of the coil body since the distal end fixed portion is only fixed to an end face of the distal end of the coil body (in other words, the distal end fixed portion does not flow towards the proximal end side along the wires at all).

### SUMMARY OF THE INVENTION

### SOLUTION TO PROBLEM

The present invention is made in view of the circumstances described above. An object of the present invention is to provide a guide wire in which a distal end fixed portion can be easily fixed to the distal end of the coil body, and the distal end fixed portion does not easily fall off from the distal end of the coil body.

The above object is achieved by the a guidewire according to claim 1. Preferred embodiments are subject-matter of dependent claims. References to embodiments throughout the description which are not under the scope of the appended claims merely represent possible exemplary executions and are therefore not part of the present invention.

The present invention relates to a guide wire comprising a core shaft, a coil body covering a distal end portion of the core shaft, having a distal end and formed of a plurality of spirally wound wires, each of the wires being a single-strand wire formed of a single strand or a multi-strand wire formed of a plurality of strands twisted together, and a distal end fixed portion in which a distal end of the distal end portion of the core shaft is fixed to the distal end of the coil body, wherein at the distal end of the coil body, the wires are tightly fixed to each other by melting, welding, or adhering without leaving a gap except for at least one gap formed between two adjacent strands of the coil body, and the distal end fixed portion formed of a solder material or a metal solder which has entered in the gap.

In other words, as viewed in a cross section, the coil body includes (i) a fixed portion in which adjacent ones of the wires are fixed to each other and (ii) at least one gap (gap portion) in which adjacent ones of strands are not fixed to each other to provide a gap between the adjacent ones of the wires in the gap portion.

In the present invention the gap (gap portion) is intentionally formed between two adjacent strands at the distal end of the coil body. The at least one gap may be formed between two adjacent wires. Further, in the case where the wire of the coil body is a multi-strand wire, the at least one gap may be formed between two adjacent strands of at least one multi-strand wire.

An embodiment of the present invention relates to the guide wire, wherein the wire is a multi-strand wire and the at least one gap is formed in the inside of at least one multi-strand wire as viewed in a cross section perpendicular to the longitudinal axis of the core shaft.

Another embodiment of the present invention relates to the guide wire, wherein the distal end portion of the coil body is curved in a certain direction, and the fixed portion faces to the opposite direction of the curvature direction of the coil body, and the gap faces to the curvature direction of the coil body. I.e., the fixed portion is located at the outward of the curve and the gap is located at the inward of the curve.

### ADVANTAGEOUS EFFECT OF THE INVENTION

In the guide wire according to the present invention, provided at the distal end of the coil body are at least one fixed portion in which the wires are fixed to each other and at least one gap portion between the strands as viewed in cross section perpendicular to the longitudinal axis of the core shaft, and a distal end fixed portion enters into the gap portion. Because both the fixed portion and the gap portion are provided at the distal end of the coil body, a risk of unwinding of the wires of the coil body can be reduced. Further, a risk can be reduced that the distal end fixed portion flows towards the proximal end side along the surfaces of all of the wires even in a case where highly flowable (highly wettable) solder materials and metal solders are used at the distal end fixed portion. Moreover, the tensile strength of the distal end fixed portion can be enhanced since the distal end fixed portion enters into the gap portion. Therefore, a risk that the distal end fixed portion falls off from the distal end of the coil body can be reduced even when external force is applied to the distal end of the coil body.

In the guide wire according to an embodiment of the present invention, the coil body is formed of a plurality of spirally wound multi-strand wires, each of which is formed of a plurality of strands twisted together, and the multi-strand wires are fixed to each other by the fixed portion, and the gap portion is formed in the inside of at least one multi-strand wire. The coil body has a degree of freedom and high flexibility because the coil body is formed of the multi-strand wires. On the other hand, the multi-strand wires of a coil body tend to unwind more easily as compared with a coil body formed with a spirally wound single wire or a plurality of spirally wound single-strand wires. However, in the guide wire according to this embodiment , the distal end of the coil body does not easily unwind even in a case where the coil body is formed of the multi-strand wires because adjacent ones of the multi-strand wires are fixed to each other by the fixed portion. Further, because the distal end fixed portion enters into the gap portion formed in the inside of a multi-strand wire, a risk that the distal end fixed portion peels off from the coil body can be reduced even in a case where the distal end of the coil body is strongly rubbed with a narrowed or obstructed segment when an operator pushes the guide wire into the narrowed or obstructed segment. As a result, a risk that the distal end fixed portion falls off from the distal end of the coil body can be further reduced.

In the guide wire according to another embodiment of the present invention, the distal end portion of the coil body is curved in a certain direction, and the fixed portion faces to the opposite direction of the curvature direction of the coil body while the gap portion faces to the curvature direction of the coil body. Therefore, the distal end fixed portion is configured to easily enter into the gap portion facing to the same direction as the curvature direction of the coil body, but not into the fixed portion facing to the opposite direction of the curvature direction of the coil body. Thereby, the distal end fixed portion does not flow towards the proximal end side in the opposite direction of the curvature direction of the coil body (in other words, the outer periphery side of the coil body) which tends to make contract with a blood vessel wall and digestive organ wall when a guide wire is inserted into a blood vessel or digestive organs. On the other hand, the distal end fixed portion flows towards the proximal end side in the same direction as the curvature direction of the coil body (in other words, the inner periphery side of the coil body) which tends not to make contact with a blood vessel wall or digestive organ wall. As a result, because the distal end fixed portion preferentially enters into the inner periphery side over the outer periphery side, a risk of damaging a wall of a blood vessel or a digestive organ wall with the outer periphery side of the coil body can be reduced when a guide wire is inserted into a blood vessel or a digestive organ.

### BRIEF DESCRIPTION OF THE DRAWINGS

Fig. 1 shows an overall view of a guide wire according to an embodiment of the present invention.
Fig. 2 shows the A-A cross section in Fig. 1.
Fig. 3 shows a perspective view of only a coil body of the guide wire shown in Fig. 1.
Fig. 4 shows an overall view of a guide wire according to a second embodiment of the present invention.
Fig. 5 shows the B-B cross section in Fig. 4.
Fig. 6 shows a perspective view of only a coil body of the guide wire shown in Fig. 4.
Fig. 7 shows a variation of Fig. 5.
Fig. 8 shows an overall view of a guide wire according to a third embodiment of the present invention.
Fig. 9 shows the C-C cross section in Fig. 8.
Fig. 10 shows an overall view of a guide wire according to a fourth embodiment of the present invention.
Fig. 11 shows the D-D cross section in Fig. 10.

### DESCRIPTION OF EMBODIMENTS

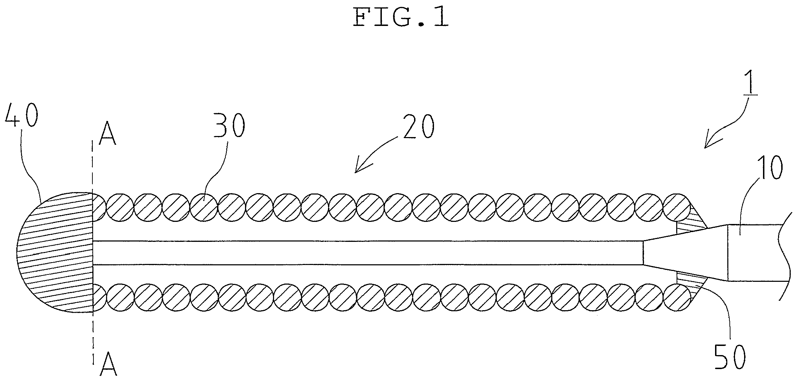

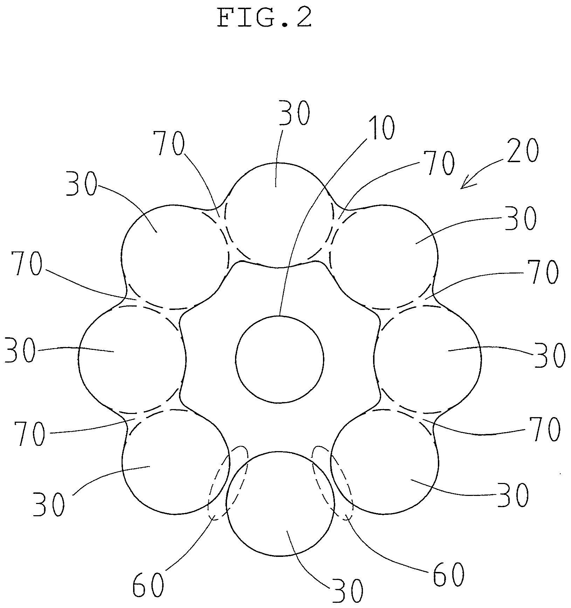

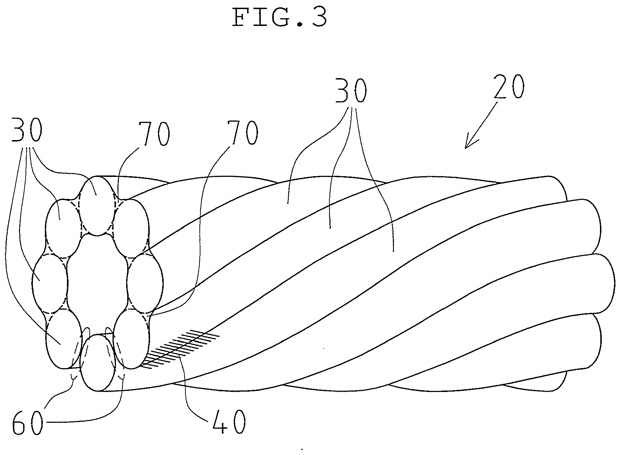

First, a guide wire 1 according to the present embodiment will be described with reference to Figs. 1 to 3. Note that the left side in Figs. 1 and 3 corresponds to the distal end side (the distal side) which is to be inserted into the body, and the right side corresponds to the proximal end side (the proximal side, the base end side) which is to be operated by an operator such as a physician. Note that Fig. 2 shows the A-A cross section in Fig. 1.

First, as shown in Fig. 1, the guide wire 1 comprises a core shaft 10, a coil body 20 covering the distal end portion of the core shaft 10 and formed of a plurality of spirally wound single-strand wires 30, each of which is formed of a single strand 30, and a distal end fixed portion 40 by which the distal end of the core shaft 10 is fixed to the distal end of the coil body 20. The proximal end of the coil body 20 is fixed to the core shaft 10 at the proximal end fixed portion 50.

As shown in Fig. 2, the coil body 20 is formed of 8 single-strand wires 30. The coil body 20 can be formed, for example, by densely twisting the 8 single-strand wires 30 on a cored bar so that they make contact with each other, and thereafter removing residual stress by a known heat treating method, and then pulling out the cored bar. Note that the number of the single-strand wires 30 of the coil body 20 is to be two or more, but is not limited to 8.

As viewed in a cross section, a fixed portion 70 in which the single-strand wires 30 are fixed to each other and at least one (one or more) gap or gap portion 60 in which the single-strand wires 30 are not fixed to each other to leave a gap between two adjacent single-strand wires 30 of the single-strand wires 30 are provided at the distal end of the coil body 20 (see Fig. 2). The fixed portion 70 is formed by melting the single-strand wires 30 by laser or electro-discharge machining in the present embodiment, but the method shall not be limited to these. The fixed portion 70 may be formed by fixing another material such as soldering, brazing or adhesive to one ore more gaps between the single-strand wires 30.

Fig. 3 shows a perspective view of the coil body 20 alone. The guide wire 1 according to the present embodiment is formed by fixing the distal end fixed portion 40 to the distal end of the coil body 20 having both the fixed portion 70 and the gap portion 60. Therefore, the distal end fixed portion 40 flows towards the proximal end side along the surfaces of the single-strand wires 30 at the gap portion 60 while the distal end fixed portion 40 does not substantially flow towards the proximal end side at the fixed portion 70 because there is no gap between the single-strand wires 30 at the fixed portion 70. Therefore, as shown in Fig. 3, the distal end fixed portion 40 enters into only the gap portion 60.

As described above, both the fixed portion 70 and the gap portion 60 are provided at the distal end of the coil body 20 in the guide wire 1 according to the present embodiment. Therefore, a risk of unwinding of the single-strand wires 30 of the coil body 20 can be reduced by providing the fixed portion 70. Further, a risk that the distal end fixed portion 40 flows towards the proximal end side along the surfaces of all of the single-strand wires 30 can be reduced even in a case where highly flowable (highly wettable) solder materials and metal solders are used at the distal end fixed portion 40. Further, since the distal end fixed portion 40 enters into the gap portion 60, the tensile strength of the distal end fixed portion 40 can be enhanced, and a risk that the distal end fixed portion 40 falls off from the distal end of the coil body 20 can be reduced even when an external force is applied to the distal end of the coil body 20.

Note that as shown in Fig. 2, two gap portions 60 and six fixed portions 70 are provided at the distal end of the coil body 20 in the present embodiment, but the configuration is not limited to this. For example, three gap portions 60 and five fixed portions 70 may be provided at the distal end of the coil body 20. Further, the same number of the gap portions 60 and the fixed portions 70 may be provided at the distal end of the coil body 20, or the number of gap portions 60 provided may be larger than that of the fixed portions 70.

Next, materials for each member of the guide wire 1 according to the present embodiment will be described, but they are not limited to these materials in particular.

The core shaft 10 can be formed of a stainless steel (SUS304, SUS316 and the like) and a superelastic alloy such as an Ni-Ti alloy.

The coil body 20 can be formed of the single-strand wires 30 which have radiopacity. Examples include gold, platinum, tungsten and alloys comprising these elements. In a case where the coil body 20 is formed of the single-strand wires 30 which have radiopacity, an operator can detect the position of the coil body 20 under radiography imaging.

The distal end fixed portion 40 and the proximal end fixed portion 50 can be formed of a solder material (an aluminum alloy solder, a silver solder, a gold solder and the like) or a metal solder (an Au-Sn alloy, an Ag-Sn alloy and the like).

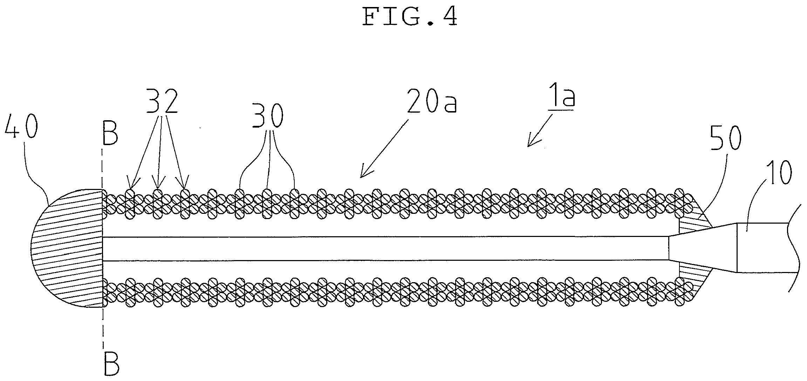

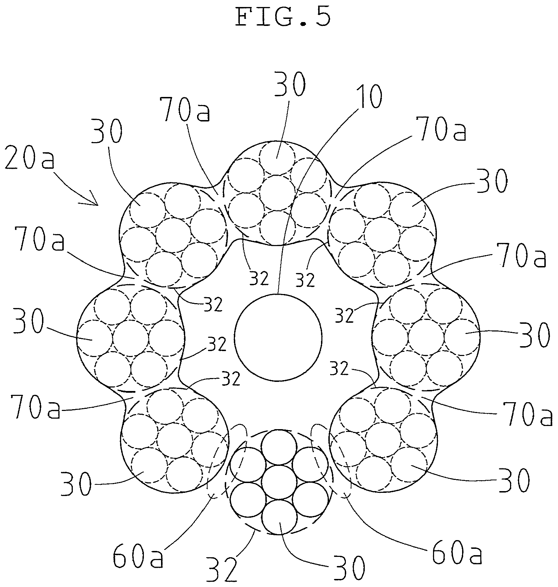

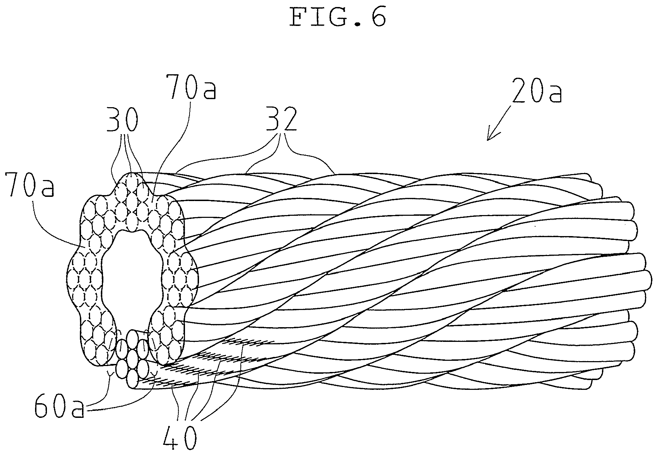

Next, a guide wire 1a according to a second embodiment will be described with reference to Figs. 4 to 7. As in Figs. 1 and 3, the left side in Figs. 4 and 6 corresponds to the distal end side (the distal side) which is to be inserted into the body, and the right side corresponds to the proximal end side (the proximal side, the base end side) which is to be operated by an operator such as a physician. Note that Fig. 5 shows the B-B cross section in Fig. 4.

Only differences from the guide wire 1 shown in Figs. 1 to 3 are described below. As shown in Figs. 4 and 5, in a guide wire 1a, a coil body 20a is formed of spirally wound eight multi-strand wires 32 each of which is formed of seven strands 30 twisted together. As viewed in a cross section, a fixed portion 70a in which the multi-strand wires 32, each of which is formed of seven strands 30 twisted together, are fixed to each other, and at least one gap or gap portion 60a in which adjacent ones of the multi-strand wires 32, each of which is formed of seven strands 30 twisted together, are not fixed to each other to leave a gap between the multi-strand wires 32 are provided at the distal end of the coil body 20a. In the present embodiment according to Figs. 4 and 5, gaps are formed between two adjacent multi-strand wires 32 and between two adjacent strands 30 of one multi-strand wire 32.

Note that the number of the multi-strand wires 32 of the coil body 20a is to be two or more, but is not limited to eight. Similarly, the number of the strands 30 of the multi-strand wire 32 is to be two or more, but shall not be limited to seven.

Fig. 6 shows a perspective view of the coil body 20a alone. The guide wire 1a according to the second embodiment is formed by fixing the distal end fixed portion 40 to the distal end of the coil body 20a having both the fixed portion 70a and the gap portion 60a. Therefore, the distal end fixed portion 40 flows towards the proximal end side at the gap portion 60a along the surfaces of the multi-strand wires 32 comprising the strands 30 while the distal end fixed portion 40 does not substantially flow towards the proximal end side at the fixed portion 70a because there is no gap between the multi-strand wires 32. Therefore, as shown in Fig. 6, the distal end fixed portion 40 enters into the gap portion 60a only.

As described above, in the guide wire 1a according to the second embodiment, both the fixed portion 70a and the gap portion 60a are provided at the distal end of the coil body 20a as in the guide wire 1. Therefore, a risk of unwinding of the multi-strand wires 32 of the coil body 20a can be reduced by providing the fixed portion 70a. Further, a risk that the distal end fixed portion 40 flows towards the proximal end side along the surfaces of all of the multi-strand wires 32 can be reduced even in a case where highly flowable (highly wettable) solder materials and metal solders are used at the distal end fixed portion 40. Moreover, the tensile strength of the distal end fixed portion 40 can be enhanced since the distal end fixed portion 40 enters into the gap portion 60a. Therefore, a risk that the distal end fixed portion 40 falls off from the distal end of the coil body 20a can be reduced even when an external force is applied to the distal end of the coil body 20a.

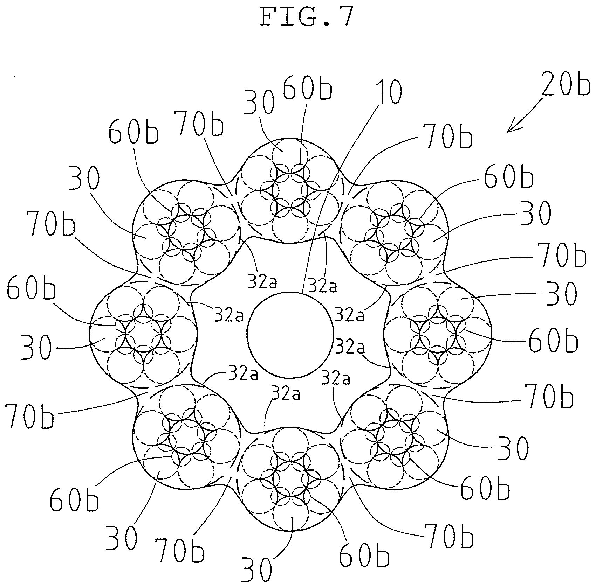

Further, Fig. 7 shows a variation of Fig. 5. In Fig. 5, two gap portions 60a are provided between adjacent multi-strand wires 32 at the distal end of the coil body 20a. In contrast, in Fig. 7, six gap portions 60b are provided in the inside of one multi-strand wire 32a at the distal end of the coil body 20b. More specifically described, as shown in Fig. 7, eight fixed portions 70b in which the multi-strand wires 32a, each of which is formed of seven strands 30 twisted together, are fixed to each other and six gap portions 60b in the inside of each of the eight multi-strand wires 32a are provided at the distal end of the coil body 20b as view in a cross section. In this variation according to Fig. 7, no gap is formed between two adjacent multi-strand wires 32a.

The coil body 20b has a degree of freedom and high flexibility because it is formed of the eight multi-strand wires 32a, but the eight multi-strand wires 32a of the coil body 20b would unwind more easily as compared with the coil body 20 of the guide wire 1. Nonetheless, as shown in Fig. 7, a risk of unwinding of the distal end of the coil body 20b can be reduced even in a case where the coil body 20b is formed of the multi-strand wires 32a because the eight multi-strand wires 32a are all fixed to each other at the eight fixed portions 70b. Further, the distal end fixed portion 40 enters into the gap portions 60b formed in the inside of the multi-strand wires 32a (in other words, the distal end fixed portion 40 does not flow towards the proximal end side along the surfaces of the multi-strand wires 32a). Therefore, even in a case where the distal end of the coil body 20b is strongly rubbed with a narrowed or obstructed segment when an operator pushes the guide wire 1a to the narrowed or obstructed segment, a risk can be reduced that the distal end fixed portion 40 which enters into the gap portions 60b peels off from the coil body 20b. As a result, a risk that the distal end fixed portion 40 falls off from the distal end of the coil body 20b can be further reduced.

Note that the number of the multi-strand wires 32a of the coil body 20b is to be two or more, but is not limited to eight. Similarly, the number of the strands 30 of the multi-strand wire 32a is to be two or more, but is not limited to seven.

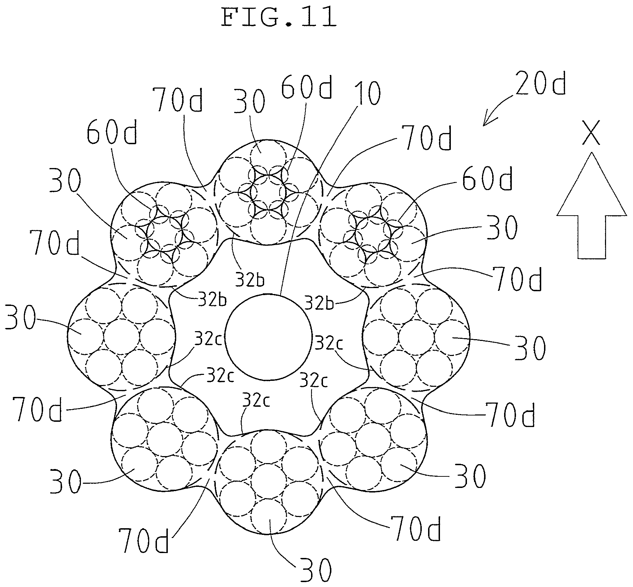

Further, the gap portions 60b are provided in the inside of each of the eight multi-strand wires 32a in Fig. 7, but the configuration is not limited to this. For example, the gap portions 60b may be provided in the inside of three multi-strand wires 32b, but not in the inside of the remaining five multi-strand wires 32c as described below (see Fig. 11). Further, six gap portions 60b are provided in the inside of one multi-strand wire 32a in Fig.7, but the configuration is not limited to this. For example, only one gap portion 60b may be provided in the inside of one multi-strand wire 32a.

Therefore, any configuration may be used as long as the coil body 20b is formed of spirally wound multi-strand wires 32a each of which is formed of 7 strands 30 twisted together, and the multi-strand wires 32a are fixed to each other by the fixed portion 70b, and the gap portion 60b is to be formed somewhere in the inside of at least one multi-strand wire 32a of the multi-strand wires 32a.

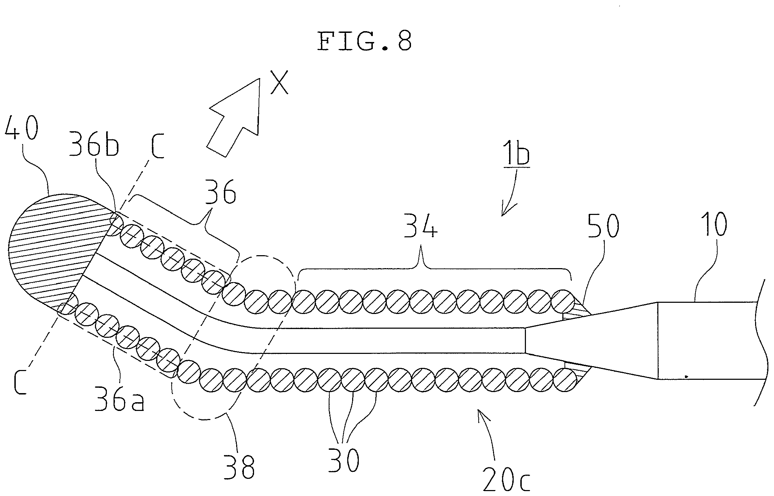

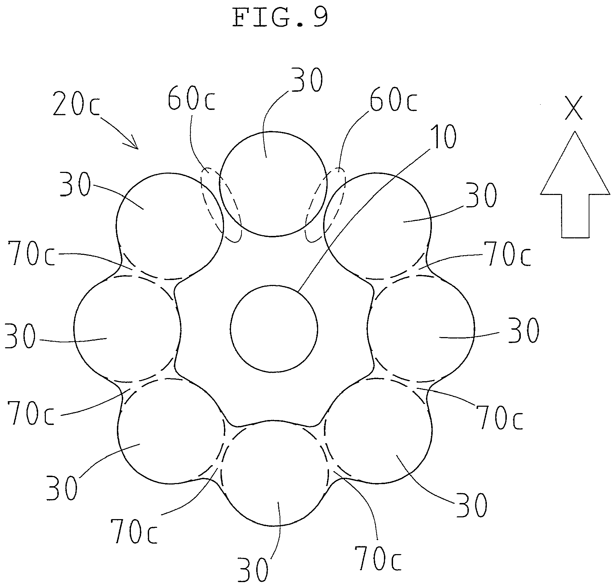

Next, the guide wire 1b according to the third embodiment will be described with reference to Figs. 8 and 9. As in Fig. 1, the left side in Fig. 8 corresponds to the distal end side (the distal side) which is to be inserted into the body, and the right side corresponds to the proximal end side (the proximal side, the base end side) which is to be operated by an operator such as a physician. Note that Fig. 9 shows the C-C cross section in Fig. 8.

Only differences from the guide wire 1 shown in Figs. 1 to 3 will be described below. As shown in Fig. 8, in a guide wire 1b, a coil body 20c comprises a first straight portion 34; a curved portion 38 located distally of the first straight portion 34 and curved in the direction of X; and a second straight portion 36 located between the curved portion 38 and the distal end fixed portion 40.

As shown in Fig. 9, a fixed portion 70c in which the single-strand wires 30 are fixed to each other and a gap portion 60c in which the single-strand wires 30 are not fixed to each other to leave a gap between adjacent ones of the single-strand wires 30 as viewed in a cross section are provided at the distal end of the coil body 20c. At this time, the fixed portion 70c faces to the opposite direction of the curvature direction (the direction of X) of the curved portion 38 of the coil body 20c, and the gap portion 60c faces to the curvature direction (the direction of X) of the curved portion 38 of the coil body 20c.

In the guide wire 1b, the distal end fixed portion 40 easily enters into the gap portion 60c facing to the same direction as the curvature direction (the direction of X) of the curved portion 38 of the coil body 20c. On the other hand, the distal end fixed portion 40 is configured to not easily enter into the fixed portion 70c facing to the opposite direction of the curvature direction (the direction of X) of the curved portion 38 of the coil body 20c. Therefore, the distal end fixed portion 40 does not flow towards the proximal end side along the surfaces of the single-strand wires 30 at an outer periphery side 36a of the second straight portion 36 which likely makes contact with a blood vessel wall or digestive organ wall, while the distal end fixed portion 40 flows towards the proximal end side along the surfaces of the single-strand wires 30 at an inner periphery side 36b of the second straight portion 36 which less likely makes contact with a blood vessel wall or digestive organ wall. As described above, since the distal end fixed portion 40 preferentially enters into the inner periphery side 36b of the second straight portion 36 as compared to the outer periphery side 36a in the guide wire 1b, rigidification due to the distal end fixed portion 40 is less significant at the outer periphery side 36a of the second straight portion 36 of the coil body 20c. As a result, a risk can be reduced that the outer periphery side 36a of the second straight portion 36 of the coil body 20c damages a blood vessel wall or digestive organ wall when the guide wire 1b is inserted into the blood vessel or digestive organ.

Note that, as shown in Fig. 9, two gap portions 60c and six fixed portions 70c are provided at the distal end of the coil body 20c in the third embodiment, but the configuration is not limited to this. For example, three gap portions 60c and five fixed portions 70c may be provided at the distal end of the coil body 20c. Further, the same number of the gap portions 60c and the fixed portions 70c may be provided at the distal end of the coil body 20c.

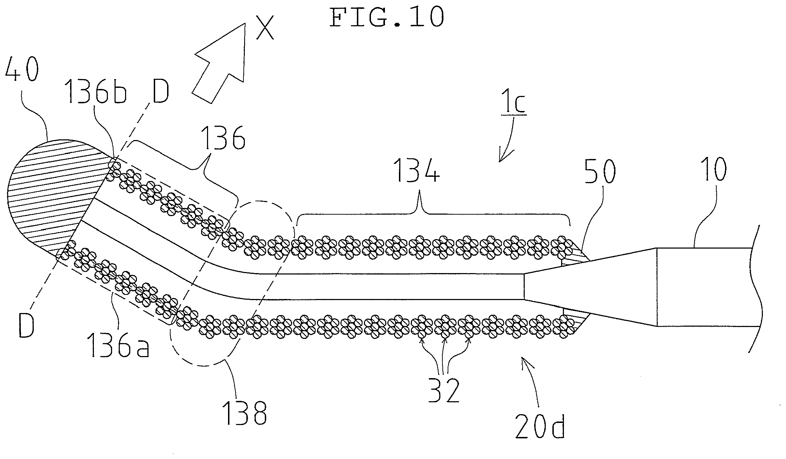

Next, a guide wire 1c according to a fourth embodiment will be described with reference to Figs. 10 and 11. As in Fig. 4, the left side in Figs. 10 corresponds to the distal end side (the distal side) which is to be inserted into the body, and the right side corresponds to the proximal end side (the proximal side, the base end side) which is to be operated by an operator such as a physician. Note that Fig. 11 shows the D-D cross section in Fig. 10.

Only differences from the guide wire 1a shown in Figs. 4 to 7 will be described below. As shown in Fig. 10, in the guide wire 1c, a coil body 20d comprises a first straight portion 134; a curved portion 138 located distally of the first straight portion 134 and curved in the direction of X; and a second straight portion 136 located between the curved portion 138 and the distal end fixed portion 40.

As shown in Fig. 11, the coil body 20d is formed of spirally wound eight multi-strand wires 32b, 32c each of which is formed of seven strands 30 twisted together. As viewed in a cross section, eight fixed portions 70d in which the multi-strand wires 32b, 32c, each of which is formed of seven strands 30 twisted together, are fixed to each other and six gap portions 60d formed in the inside of each of three multi-strand wires 32b of the eight multi-strand wires 32b, 32c are provided at the distal end of the coil body 20d. On the other hand, the gap portions 60d are not provided in the inside of five multi-strand wires 32c of the eight multi-strand wires 32b, 32c.

In Figs. 10 and 11, the multi-strand wires 32c that do not have the gap portion 60d faces to the opposite direction of the curvature direction (the direction of X) of the curved portion 138 of the coil body 20d, and the multi-strand wires 32b having the gap portion 60d faces to the same direction as the curvature direction (the direction of X) of the curved portion 138 of the coil body 20d.

In the guide wire 1c, the distal end fixed portion 40 easily enters into the gap portion 60d facing to the same direction as the curvature direction (the direction of X) of the curved portion 138 of the coil body 20d. On the other hand, the distal end fixed portion 40 is configured to not easily enter into the fixed portion 70d facing to the opposite direction of the curvature direction (the direction of X) of the curved portion 138 of the coil body 20d. Therefore, the distal end fixed portion 40 does not flow into the inside of the multi-strand wires 32c at an outer periphery side 136a of the second straight portion 136 which likely makes contact with a blood vessel wall or digestive organ wall, while the distal end fixed portion 40 flows into the inside of the multi-strand wires 32b at an inner periphery side 136b of the second straight portion 136 which less likely makes contact with a blood vessel wall or digestive organ wall. As described above, since the distal end fixed portion 40 preferentially enters into the inner periphery side 136b of the second straight portion 136 over the outer periphery side 136a in the guide wire 1c, rigidification due to the distal end fixed portion 40 is less significant at the outer periphery side 136a of the second straight portion 136 of the coil body 20d. As a result, a risk can be reduced that the outer periphery side 136a of the second straight portion 136 of the coil body 20d damages a blood vessel wall and digestive organ wall when the guide wire 1c is inserted into a blood vessel or digestive organ.

Note that in the fourth embodiment, the gap portions 60d are provided in the inside of each of three multi-strand wires 32b of the eight multi-strand wires 32b, 32c at the distal end of the coil body 20d as shown in Fig. 11, but the configuration is not limited to this. For example, the gap portion 60d may be provided in the inside of only one multi-strand wire 32b of the eight multi-strand wires 32b, 32c, but not in the inside of the remaining seven multi-strand wires 32c. Further, in Fig. 11, six gap portions 60d are provided in the inside of each of the three multi-strand wires 32b, but the configuration is not limited to this. For example, one gap portion 60d may be provided in the inside of each of the three multi-strand wires 32b. Therefore, the gap portion 60d is to be formed somewhere in the inside of at least one multi-strand wire 32b of the multi-strand wires 32b, 32c.

Note that the number of the multi-strand wires 32b, 32c of the coil body 20d is to be two or more, but shall not be limited to eight. Similarly, the number of the strands 30 of the multi-strand wires 32b, 32c is to be two or more, but shall not be limited to seven.

Further, in the guide wire 1, 1a, 1b, 1c, 1d described above, the tensile strength of the distal end fixed portion 40 may be improved by grinding the distal end of the coil body 20, 20a, 20b, 20c, 20d before fixing the distal end fixed portion 40 to the distal end of the coil body 20, 20a, 20b, 20c, 20d to increase the contact area between the fixed portion 70, 70a, 70b, 70c, 70d and the distal end fixed portion 40. This can further reduce a risk that the distal end fixed portion 40 falls off from the distal end of the coil body 20, 20a, 20b, 20c, 20d even in a case where an external force is applied to the distal end of the coil body 20, 20a, 20b, 20c, 20d.

As described above, in the guide wires 1, 1a, 1b, 1c, 1d, both the fixed portion 70, 70a, 70b, 70c, 70d and the gap portion 60, 60a, 60b, 60c, 60d are provided at the distal end of the coil body 20, 20a 20b, 20c, 20d. This can reduce a risk of unwinding of the single-strand wires 30 or the multi-strand wires 32, 32a, 32b, 32c of the coil body 20, 20a, 20b, 20c, 20d. Further, a risk that the distal end fixed portion 40 flows towards the proximal end side along the surfaces or insides of the single-strand wires 30 or the multi-strand wires 32, 32a, 32b, 32c can be reduced even in a case where highly flowable (highly wettable) solder materials and metal solders are used at the distal end fixed portion 40. Further, the tensile strength of the distal end fixed portion 40 can be enhanced since the distal end fixed portion 40 enters into the gap portion 60, 60a, 60b, 60c, 60d. Therefore, a risk that the distal end fixed portion 40 falls off from the distal end of the coil body 20, 20a, 20b, 20c, 20d can be reduced even in a case where an external force is applied to the distal end of the coil body 20, 20a, 20b, 20c, 20d.

### DESCRIPTION OF SYMBOLS

- 1, 1a, 1b, 1c: Guide wire
- 10: Core shaft
- 20, 20a, 20b, 20c, 20d: coil body
- 30: Single-strand wire, Strand
- 32, 32a, 32b, 32c: Multi-strand wire
- 34, 134: First straight portion
- 36, 136: Second straight portion
- 36a, 136a: Outer periphery side
- 36b, 136b: Inner periphery side
- 38, 138: Curved portion
- 40: Distal end fixed portion
- 50: Proximal end fixed portion
- 60, 60a, 60b, 60c, 60d: Gap portion
- 70, 70a, 70b, 70c, 70d: Fixed portion

## Claims

1. A guide wire (1, 1a, 1b, 1c) comprising:
a core shaft (10),
a coil body (20, 20a, 20b, 20c, 20d) covering a distal end portion of the core shaft (10), having a distal end and formed of a plurality of spirally wound wires (30, 32), each of the wires (30, 32) being a single-strand wire formed of a single strand or a multi-strand wire formed of a plurality of strands twisted together, and
a distal end fixed portion (40) in which a distal end of the core shaft (10) is fixed to the distal end of the coil body (20, 20a, 20b, 20c, 20d),
**characterized in that**
as viewed in a cross section perpendicular to the longitudinal axis of the core shaft (10), the distal end of the coil body (20, 20a, 20b, 20c, 20d) includes (i) a fixed portion (70) in which adjacent ones of the wires (30, 32) are fixed to each other by melting, welding, or adhering, and (ii) at least one gap portion (60) in which adjacent ones of strands (30, 32a, 32b, 32c) are not fixed to each other by melting, welding, or adhering to provide a gap between the adjacent ones of the wires (30, 32) in the gap portion (60), and
the distal end fixed portion (40) is formed of a solder material or a metal solder which has entered the gap portion (60).

2. The guide wire according to claim 1, **characterized in that** each of the wires (30) is a single-strand wire.

3. The guide wire according to claim 1, **characterized in that** each of the wires (32, 32a, 32b, 32c) is a multi-strand wire.

4. The guide wire according to any one of claims 1 to 3, **characterized in that** the at least one gap is formed between two adjacent wires (30, 32).

5. The guide wire according to claim 3, **characterized in that** the at least one gap is formed between two adjacent strands of at least one multi-strand wire.

6. The guide wire according to claim 3, **characterized in that** the at least one gap is formed in the inside of at least one multi-strand wire, as viewed in a cross section.

7. The guide wire according to any one of claims 1 to 6, **characterized in that** the distal end portion of the coil body (20c, 20d) is curved in a certain direction, the gap faces to the curvature direction of the coil body.

## Patentansprüche

1. Führungsdraht (1, 1a, 1b, 1c) mit:
einem Kernschaft (10),
einem Wicklungskörper (20, 20a, 20b, 20c, 20d), der einen distalen Endabschnitt des Kernschafts (10) ummantelt, ein distales Ende hat und aus einer Vielzahl von schraubenförmig gewickelten Drähten (30, 32) gebildet ist, von denen jeder ein einsträngiger Draht, der aus einem einzelnen Strang gebildet ist, oder ein mehrsträngiger Draht ist, der aus einer Vielzahl von miteinander verdrillten Drähten gebildet ist, und
einem an dem distalen Ende befestigten Abschnitt (40), in dem ein distales Ende des Kernschafts (10) an dem distalen Ende des Wicklungskörpers (20, 20a, 20b, 20c, 20d) befestigt ist,
**dadurch gekennzeichnet, dass**
in einem Querschnitt senkrecht zur Längsachse des Kernschafts (10) gesehen, das distale Ende des Wicklungskörpers (20, 20a, 20b, 20c, 20d) (i) einen Befestigungsabschnitt (70), in dem aneinandergrenzende Drähte (30, 32) miteinander verschmolzen, verschweißt oder verklebt sind, und (ii) wenigstens einen Lückenabschnitt (60) aufweist, in dem aneinandergrenzende Stränge (30, 32a, 32b, 32c) nicht miteinander verschmolzen, verschweißt oder verklebt sind, um zwischen den aneinandergrenzenden Drähten (30, 32) in dem Lückenabschnitt (60) eine Lücke zu schaffen, und
der an dem distalen Ende befestigte Abschnitt (40) aus einem in den Lückenabschnitt (60) eingedrungenen Lotmaterial oder Metalllot gebildet ist.

2. Führungsdraht nach Anspruch 1, **dadurch gekennzeichnet, dass** jeder der Drähte (30) ein einsträngiger Draht ist.

3. Führungsdraht according to claim 1, **dadurch gekennzeichnet, dass** jeder der Drähte (32, 32a, 32b, 32c) ein mehrsträngiger Draht ist.

4. Führungsdraht nach einem der Ansprüche 1 bis 3, **dadurch gekennzeichnet, dass** die wenigstens eine Lücke zwischen zwei aneinandergrenzenden Drähten (30, 32) ausgebildet ist.

5. Führungsdraht nach Anspruch 3, **dadurch gekennzeichnet, dass** die wenigstens eine Lücke zwischen zwei aneinandergrenzenden Strängen wenigstens eines mehrsträngigen Drahts ausgebildet ist.

6. Führungsdraht nach Anspruch 3, **dadurch gekennzeichnet, dass** die wenigstens eine Lücke, in einem Querschnitt gesehen, im Innern wenigstens eines mehrsträngigen Drahts ausgebildet ist.

7. Führungsdraht nach einem der Ansprüche 1 bis 6, **dadurch gekennzeichnet, dass** der distale Endabschnitt des Wicklungskörpers (20c, 20d) in eine bestimmte Richtung gekrümmt ist, und die Lücke auf der Krümmungsrichtungsseite des Wicklungskörpers liegt.

## Revendications

1. Fil-guide (1, 1a, 1b, 1c) comprenant :
un arbre central (10),
un corps de bobine (20, 20a, 20b, 20c, 20d) recouvrant une partie d'extrémité distale de l'arbre central (10), ayant une extrémité distale et formé avec une pluralité de fils enroulés en spirale (30, 32), chacun des fils (30, 32) étant un fil à brin unique formé avec un seul brin ou un fil à plusieurs brins formé avec une pluralité de brins torsadés ensemble, et
une partie fixe d'extrémité distale (40) dans laquelle une extrémité distale de l'arbre central (10) est fixée à l'extrémité distale du corps de bobine (20, 20a, 20b, 20c, 20d),
**caractérisé en ce que** :
comme observé dans une coupe transversale perpendiculaire à l'axe longitudinal de l'arbre central (10), l'extrémité distale du corps de bobine (20, 20a, 20b, 20c, 20d) comprend (i) une partie fixe (70) dans laquelle des fils adjacents des fils (30, 32) sont fixés entre eux par fusion, soudage ou collage et (ii) au moins une partie d'espace (60) dans laquelle des brins adjacents des brins (30, 32a, 32b, 32c) ne sont pas fixés entre eux par fusion, soudage ou collage pour fournir un espace entre les fils adjacents des fils (30, 32) dans la partie d'espace (60), et
la partie fixe d'extrémité distale (40) est formée avec un matériau de soudure ou une soudure métallique qui a pénétré dans la partie d'espace (60).

2. Fil-guide selon la revendication 1, **caractérisé en ce que** chacun des fils (30) est un fil à brin unique.

3. Fil-guide selon la revendication 1, **caractérisé en ce que** chacun des fils (32, 32a, 32b, 32c) est un fil à plusieurs brins.

4. Fil-guide selon l'une quelconque des revendications 1 à 3, **caractérisé en ce que** le au moins un espace est formé entre deux fils (30, 32) adjacents.

5. Fil-guide selon la revendication 3, **caractérisé en ce que** le au moins un espace est formé entre deux brins adjacents d'au moins un fil à plusieurs brins.

6. Fil-guide selon la revendication 3, **caractérisé en ce que** le au moins un espace est formé à l'intérieur d'au moins un fil à plusieurs brins, comme observé en coupe.

7. Fil-guide selon l'une quelconque des revendications 1 à 6, **caractérisé en ce que** la partie d'extrémité distale du corps de bobine (20c, 20d) est incurvée dans une certaine direction, l'espace faisant face à la direction de courbure du corps de bobine.
